# EUROPEAN PATENT SPECIFICATION

(11) **EP 1 871 745 B1**
(45) Date of publication and mention of the grant of the patent: **20.10.2010**
(21) Application number: 06733056.3
(22) Date of filing: 07.04.2006
(51) Int. Cl.: C07D 233/68, C07D 233/54

(54) **PREPARATION OF 2-SUBSTITUTED 4-CHLORO-5-FORMYLIMIDAZOLES BY VILSMEIER REACTION OF THE CONDENSATION PRODUCT OF GLYCINE AND AN IMIDO ESTER WITH A FORMAMIDE IN THE PRESENCE OF A TRIFLATE (TRIFLUORMETHANESULPHONATE) CATALYST**
HERSTELLUNG VON 2-SUBSTITUIERTEN 4-CHLOR-5-FORMYLIMIDAZOLEN DURCH VILSMEIER-REAKTION DES KONDENSATIONSPRODUKTS VON GLYCIN UND EINEM IMIDOESTER MIT EINEM FORMAMID IN GEGENWART EINES TRIFLAT- (TRIFLUORMETHANSULFONAT-) KATALYSATORS
PRÉPARATION DE 4-CHLORO-5-FORMYLIMIDAZOLES 2-SUBSTITUTÉS PAR REACTION VILSMEIER DU PRODUIT DE CONDENSATION DE GLYCINE ET UN IMIDO ESTER AVEC UNE FORMAMIDE EN PRÉSENCE D'UN CATALYSEUR TRIFLAT (TRIFLUORMETHANESULFONATE)

(30) Priority: 15.04.2005 EP 05103014; 15.04.2005 US 671471 P
(43) Date of publication of application: 02.01.2008
(73) Proprietor: Dishman Pharmaceuticals and Chemicals Ltd., Navrangpura, Ahmedabad 380 009 (IN)
(72) Inventor: RAJNIKANT VYAS, Janmejay, London Greater London W1H 5LP (GB); SATYA VARMA NIDADAVOLU, Venkata, Ahmedabad 380 058 (IN); PRAKASH SINGH, Anand, Ahmedabad 382 340 (IN)
(74) Representative: Swinkels, Bart Willem
(86) International application number: PCT/NL2006/050077
(87) International publication number: WO 2006/110037

(56) References cited:
- US-A- 5 696 272
- AMBALAVANAN ET AL: "Crystal Structures of two Imidazole Derivatives" MIL. CRYST. LIQ. CRYST. SCI. TECHNOL. SECT. A, vol. 393, 2003, pages 75-82, XP009054531
- DAVOOD ET AL: "Synthesis and calcium channel antagonist activity of nifedipine analogues containing 4(5)-chloro-2-methyl-5(4)-imidazolyl substituent" BOLLETTINO CHIMICO FARMACEUTICO, vol. 140, no. 6, 2001, pages 381-386, XP009054522 MILANO, IT
- KEENAN ET AL: "Potent nonpeptide angiotensin II receptor antagonists. 2. 1-(Carboxybenzyl)imidazole-5-acrylic acids" JOURNAL OF MEDICINAL CHEMISTRY, vol. 36, no. 13, 1993, pages 1880-1892, XP002346837
- SHILCRAT ET AL: "A New Regioselective Synthesis of 1,2,5-Trisubstituted 1H-Imidazoles and Its Application to the Development of Eprosartan" JOURNAL OF ORGANIC CHEMISTRY, vol. 62, no. 24, 1997, pages 8449-8454, XP002346838

## Description

### FIELD OF THE INVENTION

The invention relates to a process of producing formylimidazoles, in particular a new process for the preparation of 2-substituted 5-formylimidazoles, in particular 2-butyl-5-formylimidazole. The invention also relates to an improved process for the preparation of 2-substituted 4-chloro-5-formylimidazoles, especially 2-butyl-4-chloro-5-formylimidazole.

### BACKGROUND OF THE INVENTION

Formylimidazoles are important intermediates for pharmaceutical active ingredients, for example diuretics and antihypertensive agents.

In the art 2-substituted 5-formylimidazoles are produced on a commercial scale via a 2-substituted 4-hydroxymethylimidazole intermediate. According to Y.-J. Shi et al. "A practical synthesis of 2-butyl-4(5)-chloro-5(4)-hydroxymethyl-1Himidazole", Synthetic Communications 23 (1993) p. 2623 - 2630, the intermediate 2-butyl-4-hydroxymethylimidazole can be prepared by reacting methyl pentanimidate with 1,3-dihydroxyacetone at elevated reaction temperature and at high NH₃ pressure. Although extremely high pressures of about 28 bar are reported, the reaction pressure can be reduced to an acceptable level of 3 bar (at 70 °C) in the presence of 10 equivalents of ammonia if the proper solvent is chosen, for instance from diisopropyl ether, toluene and methanol. Methanol showed to be particularly suitable, resulting in 2-butyl-4-hydroxymethylimidazole with 79 % yield, attributed to the great solubility of NH₃ in methanol.

The second step then involves the oxidation of the hydroxymethylimidazole to the corresponding formylimidazole. According to US 5,336,779 the oxidation of a hydroxymethylimidazole can be performed with a reagent containing a heavy metal, e.g. manganese dioxide or nitric acid, but more beneficially, with a noble metal catalyst such as platinum-bismuth, platinum black, platinum or palladium on activated carbon, while passing in oxygen. Alternatively, the noble metal catalyst can be used in combination with hydrogen peroxide according to US 6,040,457.

However, the synthesis of formylimidazoles via hydroxymethylimidazole does not comply with the requirements of a large-scale industrial process, because of the necessity of high pressure to establish imidazole ring closure in the first step. Moreover, although Y.-J. Shi et al. report acceptable yields of about 71 % for chloro-5-formylimidazole prepared from the hydroxymethylimidazole intermediate, 2-substituted 5-formylimidazole is obtained from the same intermediate through oxidation with less than 40 % yield. This is probably related to overoxidation of the intermediate compound.

For these reasons inexpensive alternative synthesis routes are searched, therewith avoiding the need for 2-substituted 5-hydroxymethylimidazole compounds. For 2-substituted 5-chloroformylimidazole this has resulted in the synthesis route taught in US 5,696,272, wherein glycine is reacted with an imido ester such as methyl pentanimidate, and the intermediate compound thus obtained is then converted to a 2-substituted 5-chloroformylimidazole by a Vilsmeier reagent which is composed of a chlorinating agent and a formamide. Both steps are conveniently performed in a one-pot synthesis, without the need to isolate the intermediate compound before subsequent ring closure and chlorination.

P. Ambalavanan et al. "Crystal structures of two imidazole derivatives" Mol. Cryst. Liq. Cryst. vol. 393 (2003) 75 - 82 describes the synthesis of 2-n-butyl-5-chloro-3H-imidazole-4-carbaldehyde (BCIC) from glycine and methyl pentanimidate using POCl₃. A 55% yield is reported. Similarly, A. Davood et al. "Synthesis and calcium channel antagonist activity of nifedipine analogues containing 4(5)-chloro-2-methyl-5(4)-imidazolyl substituent" Bollettino Chimico Farmaceutico vol. 140, no. 6 (2001) 381-386 teaches the synthesis of 4(5)-chloro-2-methylimidazole-5 (4)-carboxaldehyde from glycine using POCl₃ with a success rate of 14 %.

Unfortunately, despite all efforts to produce 2-substituted 5-chloroformylimidazoles more economically, yields and purity still stay behind in comparison to the high-pressure process according to J. Shi et al.. For 2-butyl 5-chloroformylimidazole a yield of 62 %, based on glycine, and a purity of about 85 % were reported in example 4 of US 5,696,272. To a large extent example 4 of US 5,696,272 is repeated in example 2 and comparative example 2 attached to the present specification. Other prior arts report even lower yields.

### DESCRIPTION OF THE INVENTION

It is an object of the invention to provide 2-substituted 4-chloro-5-formylimidazoles in higher yields and with high purity levels compared to methods existing in the art, and to simplify the synthesis route leading to these compounds.

It has been found that the yield of the production of 2-substituted 4-chloro-5-formylimidazoles, sometimes also referred to as 2-substituted 5-chloroimidazole-4-carbaldehydes, can be increased with another 15 % to over 70 %, based on glycine, by performing the Vilsmeier reaction in the synthesis route according to US 5,696,272 in the presence of a triflate catalyst. The use of such a catalyst also enhances the purity of the final product to more than 99.5 %, comparable to a commercial-grade compound, far better than achieved in the art. The high purity is realised without any additional recrystallisation steps.

The invention relates to process for the preparation of a 2-substituted 4-chloro-5-formylimidazole of the formula: 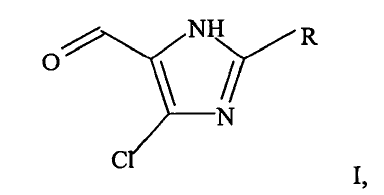 in which R is alkyl, alkenyl, cycloalkyl, arylalkyl or aryl, wherein glycine is reacted with an imido ester of the formula: 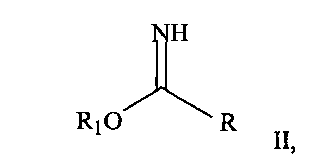 in which R has the meaning recited above, and R₁ is alkyl, to give a compound of the formula: 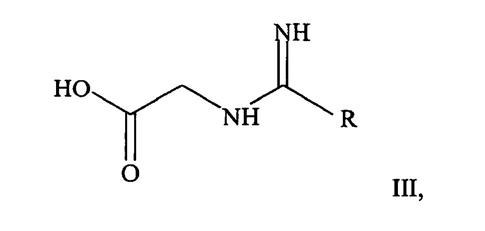 which is then subjected to a Vilsmeier reaction using a chlorinating agent and a formamide of the formula: 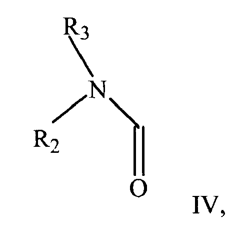 in which R₂ and R₃ are identical or different and each is a (C₁ - C₄) alkyl,
wherein said Vilsmeier reaction is performed in the presence of a triflate catalyst.

In formulae I, II, III and IV, the general substituents R, R₁, R₂ and R₃ have the following meanings:

An alkyl group is taken to mean a straight-chain or branched (C₁-C₆)-alkyl group, in particular methyl, ethyl, n-propyl, isopropyl, n-butyl, isobutyl, t-butyl, pentyl and its isomers, or hexyl and its isomers. A preferred alkyl group for R is the n-butyl group. A preferred alkyl group for R₁ is a (C₁-C₄)-alkyl group, particularly preferably methyl.

An alkenyl group is taken to mean a straight-chain or branched (C₁-C₆)-alkenyl group, in particular 1-propenyl, 2-propenyl, 1-butenyl, 2-butenyl, 3-butenyl, pentenyl and its isomers, or hexenyl and its isomers. A preferred alkenyl group is 2-butenyl or 3-butenyl.

Cycloalkyl is expediently taken to mean cyclopropyl, cyclobutyl, cyclopentyl or cyclohexyl.

An arylalkyl group expediently has the meaning phenyl-(C₁-C₆)-alkyl, preferably benzyl. Aryl correspondingly has the preferred meaning of phenyl.

The aryl group can have one or more substituents, such as, (C₁-C₄)-alkyl, alkoxy, halo, nitro or amino, on its aromatic nucleus.

The term halo expediently includes chlorine, bromine or iodine, preferably chlorine.

It is to be noted that tautomers, in particular 5-chloro-4-formylimidazoles and analogues are also encompassed by the above description.

The reaction of the imido ester with glycine is preferably performed at a pH between 4 and 12, and at a temperature between - 20 and 80 °C. The glycine is customarily present suspended in a suitable solvent, such as an aliphatic alcohol such as methanol or ethanol, optionally mixed with water. The imido ester can be added in the form of a solution in an inert solvent, such as toluene, chlorobenzene, or an aliphatic alcohol such as methanol. The reaction partners in the first stage are preferably used stoichiometrically.

After a reaction time expediently of 2 hours to 48 hours, the resulting compound of the general formula III can be isolated from the reaction mixture in a manner known to those skilled in the art, but preferably is not isolated and instead is further reacted directly in the Vilsmeier reaction.

The Vilsmeier reagent comprises a chlorinating agent, preferably selected from the group consisting of phosphorus oxychloride, thionyl chloride, phosgene or phosgene-releasing compounds, phosphorus trichloride or phosphorus pentachloride. A preferred chlorinating agent is phosphorus oxychloride.

The Vilsmeier reagent further comprises a formamide of the general formula IV. Expediently the molar ratio of chlorinating agent to formamide is between 1 to 1 and 4 to 1. The preferred formamide is N,N-dimethylformamide. The Vilsmeier reagent is preferably used in excess, serving as a solvent at the same time. However, it is also possible to add an inert solvent such as toluene, chlorobenzene or xylene. The reaction temperature for the Vilsmeier reaction is preferably between 60 and 200 °C.

The Vilsmeier reaction is performed in the presence of a triflate catalyst, more formally known as a trifluoromethanesulfonate catalyst. Other perfluoroalkanesulfonate catalyst can also be used. It is preferably a lanthanide(III) or group IV metal trifluoromethanesulfonate, more preferably the metal cation is copper(II), cerium(IV) or lanthanum(III). The catalyst is preferably present in an amount between 0.1 and 10 wt% based on glycine, more preferably between 0.2 and 8 wt%, most preferably between 0.5 and 5 wt%, based on the weight of glycine.

The final product is a 2-substituted 4-chloro-5-formylimidazole. It is preferably 2-butyl-4-chloro-5-formylimidazole (BCFI), obtained from reacting glycine with methyl pentanimidate, wherein (pentanimidoylamino)acetic acid is the intermediate compound converted in the Vilsmeier reaction. The 2-substituted 4-chloro-5-formylimidazole is typically produced by the process according to the invention with a yield of about 70 - 75 %, based on glycine, and with a purity of more than 99 %, preferably even more than 99.5 % as determined by HPLC.

It has also been found that the preparation of a 2-substituted 4-chloro-5-formylimidazole according to US 5,696,272 can start from a simpler and therefore economically attractive nitrile having formula R-C≡N rather than from a commercial-grade imido ester, and in which R has the meaning mentioned above. Furthermore, the reaction of R-C≡N to the imido ester intermediate can be carried out in the remaining established one-pot synthesis, without the requirement of an isolation or purification of the imido ester thus prepared. This makes the additional step even more suitable for industrial scale. Preferably R-C≡N is valeronitrile, leading to the final product 2-butyl-4-chloro-5-formylimidazole.

The invention thus also relates to a process for the preparation of a 2-substituted 4-chloro-5-formylimidazole, wherein said imido ester of formula (II) is prepared by reacting a nitrile having formula R-C≡N, preferably valeronitrile, with methanol in the presence of hydrochloric acid gas, followed by a treatment with ammonia.

This reaction is preferably performed at a temperature between -20 and 10 °C, and HCl gas is blown through the reaction temperature for a time between 5 - 24 hours. It is preferred to add another amount of methanol afterwards, and bring the reaction mixture in a methanolic ammonia solution at a pH between 7 and 11, while the temperature is preferably maintained at 0 - 50 °C. The reaction is completed within 10 hours. The precipitated salts are removed by filtration and washed with an aliphatic alcohol, preferably methanol, and the filtrate could be concentrated in a manner known to those skilled in the art. However, the imido ester is preferably not isolated and instead is further reacted directly with glycine.

It has also been found that a 2-substituted 5-formylimidazole, sometimes also referred to as 2-substituted imidazole-4-carbaldehyde, can conveniently be prepared from the corresponding 4-chloro-5-formylimidazole by applying a hydrodehalogenation step. Yields are observed higher than 50 %, based on glycine, far better than if prepared from the hydroxymethylimidazole as taught in the art. This is surprising, given the fact that this new method of producing 2-substituted 5-formylimidazole involves a more elaborate synthesis route, including an additional step of dechlorination. The method has the advantage that it can be performed as a one-pot synthesis, starting from simple and inexpensive compounds such as valeronitrile and glycine.

The invention thus also relates to a process for the production of a 2-substituted 5-formylimidazole of the formula: 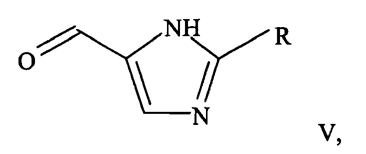 in which R is alkyl, alkenyl, cycloalkyl, arylalkyl or aryl, by performing the steps as described before, to obtain the 2-substituted 4-chloro-5-formylimidazole of formula I, and subjecting said 2-substituted 4-chloro-5-formylimidazole to hydrodehalogenation in the presence of a noble metal catalyst.

The preferred 2-substituted 5-formylimidazole is 2-butyl 5-formylimidazole, prepared from 2-butyl-4-chloro-5-formylimidazole.

Hydrodehalogenation is performed in the presence of a catalyst comprising a noble metal (including the metallic form as well as the form of a salt, oxide or the like) selected from the group consisting of platinum, palladium and gold. In particular, platinum and palladium are suited for practical use, most preferably palladium. These noble metals may be used in combination with bismuth, cerium, lead, indium or the like as a second component.

The noble metal catalyst is used as such or, when necessary, in the form supported on a carrier such as active carbon, silica or alumina. The noble metal catalyst is preferably palladium on carbon, palladium being present in an amount between 5 and 15 wt%, based on the weight of 2-butyl-4-chloro-5-formylimidazole

The hydrodehalogenation is preferably preformed in the presence of an aliphatic alcohol such as methanol, and triethylamine, preferably in an amount of 1 - 20 wt%, more preferably 5 - 15 wt%, based on the total reaction mixture, and a 2 - 10 kg, even more preferably 4 - 5 kg hydrogen pressure, and at a preferred temperature of 0 - 50 °C, more preferably 15 - 30 °C. The noble metal catalyst is preferably present in an amount of 0.1 - 2 wt%, more preferably 0.5 -1 wt%.

In a preferred embodiment the 2-substituted 5-formylimidazole is produced from a 2-substituted 4-chloro-S-formylimidazole that is produced by the process according to the invention, i.e. wherein the Vilsmeier reaction is performed in the presence of a triflate catalyst.

In an even more preferred embodiment the 2-substituted 5-formylimidazole is produced from R-C≡N, wherein R has the meaning cited above, according to the aforementioned process. It is thus possible to produce 2-substituted 5-formylimidazole without any intermediate isolation and purification steps according to an advantageous one-pot synthesis route, thereby reaching yields of more than 50 %, based on glycine, and a purity of more than 98 %, as determined by HPLC.

### EXAMPLES

### Example I - Preparation of methyl pentanimidate from valeronitrile

100 g (1.20 mol) valeronitrile was charged in 58 ml of methanol and cooled to -5 to -10 °C. HCl gas was slowly passed through the solution for 15-18 hrs. Nitrogen pressure of 1.5 to 2.0 kg /cm² was applied for 14 hrs at 0 - 15 °C, followed by the addition of 55 ml methanol and stirring for another 60 min.

The reaction mass was then transferred to a methanolic ammonia solution (12-15 wt%) and stirred for 3 hrs at 20 - 30 °C, while maintaining the pH at 8 - 9. Precipitated material was then filtered and washed with 25 ml of methanol. The filtrate was concentrated until complete removal of methanol by distillation under reduced pressure (650-700 mm Hg) at a temperature not exceeding 90 °C. Upon cooling the intermediate (methyl pentanimidate) was obtained with 95% purity, yield 140 g (1.15 mol; 96 %) as a semi-solid.

### Comparative example 1

### a. Preparation of 2-butyl-4-hydroxymethylimidazole (BHI)

140 g (1.15 mol) methyl pentanimidate prepared according to the method of example 1, 95 % purity and 84 g (0.93 mol) 1,3-dihydroxyacetone were charged in 47 ml of isopropyl alcohol and cooled to 0 - 5 °C. Ammonia gas was passed through at a pressure of 13 - 16 kg/cm² at a temperature of 65 - 70°C while stirring for 6 hrs. The reaction mixture was cooled to room temperature and the ammonia pressure was released. The reaction mixture was then subsequently heated to 55 - 60 °C, transferred to 350 ml of deionised water, stirred for 30 min, and cooled to 0 - 5 °C. The cold mixture was stirred for another 5 hrs at 0 - 5 °C, filtered and washed with 25 ml of deionised water.

Crude material was recrystallised by dissolution into 100 ml of acetonitrile at 65 - 70 °C. The clear solution was then cooled to 0 - 5 °C and stirred for another 2 hrs. Finally, the solid material was filtered off, washed with 12.5 ml of acetonitrile and dried under vacuum at 30 - 40 °C for 6 - 8 hrs. The yield of 2-butyl-4-hydroxymethylimidazole obtained was 87 g (0.55 mol ; 48 %) with a purity of 97% by HPLC.

### b. Preparation of 2-butyl-5-formylimidazole from BHI

920 ml of 2.5% sodium hydroxide solution, 6.8 g of 5% platinum on carbon, 1.05 g bismuth sulfate and 85 g (0.54 mol, 97% purity by HPLC) of 2-butyl-4-hydroxymethylimidazole were mixed at ambient temperature and then heated to 53 - 55 °C. 50% hydrogen peroxide solution (46 g) was added in 2 - 3 hrs at 58 - 62°C.. While stirring for another 90 min oxygen was passed through the solution to further increase the conversion to at least 85%. The conversion was monitored by in-process analysis.

When the conversion was complete, the reaction mixture was cooled to 50 - 55 °C, followed by filtration to remove the platinum on carbon. The filtered catalyst was washed with 25 ml of deionised water. The remaining reaction mixture was cooled to 10 - 15 °C and the pH adjusted to 7.5 - 8.0 using 50% sulfuric acid, and stirred for another 3 hrs before filtering and washing with 2 x 50 ml of chilled deionised water (10 °C).

The crude material was purified by recrystallisation from dichloromethane and hexane to give a yield of 51.5 g (0.33 mol, 61% ) of 2-butyl-5-formylimidazole with 98% purity.

### Example 2 - Preparation of BCFI from methyl pentanimidate using a triflate catalyst.

50 g (0.666 mol) of glycine was added to freshly prepared methanolic sodium hydroxide solution (sodium hydroxide 26.64 g (0.666 mol) in 250 ml of methanol) at 0°C and stirred for another 15 min. 80 g (0.70 mol) of the methyl pentanimidate prepared according to example 1 was added over a period of 10 - 15 min to the above suspension at 0 - 5 °C and stirring was continued for 16 hrs at room temperature. The solvent was then distilled under vacuum below 50°C.

500 ml of toluene was added to the above reaction mass, followed by 0.25 g of Copper(II) trifluoromethanesulfonate. Then 320 g (2.08 mol) of phosphorous oxychloride was added to this reaction mixture in 60 min, followed by 150 g (2.05 mol) N,N-dimethylformamide in 2 hrs. The reaction mixture was heated to 100 °C and stirred for 2 hrs, then cooled to 30 °C and quenched in 260 ml of cooled deionised water (temperature below 25 °C). 30 g of filter aid (hi-flow) was added and the pH adjusted to 1.2 using 440 ml of 30% aqueous sodium hydroxide solution.

It was then filtered and washed with 100 ml of toluene, and the layers were separated. The toluene layer was washed twice with deionised water (400 ml each time). 8 g of activated carbon was added to the toluene and the layer was stirred for 30 min at 30 - 35 °C, followed by filtration and washing with 100 ml of toluene. All toluene layers were combined and concentrated to 50 vol% under vacuum below 55 °C. The concentrated toluene solution was then cooled to 0 - 5 °C and stirred for 2 hrs, followed by filtration of the precipitated product and washing with 25 ml of chilled toluene, to yield a wet material, which upon drying at 50 - 55 °C to constant weight resulted in 89 g (0.47 mol) of crystalline 2-butyl-4-chloro-5-formylimidazole (yield 71 %). The analysis of this product by HPLC gave a purity of 99.8%, confirmed by IR. Melting range 95 - 99 °C.

### Comparative example 2 - Preparation of BCFI without triflate catalyst

The procedure reported in example 2 was exactly repeated, with the only difference that phosphorous oxychloride was now added in the absence of Copper(II) trifluoromethanesulfonate. All other steps, including reaction times and temperatures, were in accordance with those mentioned in example 2.

It resulted in a wet material, which upon drying at 50-55 °C to constant weight resulted in 82.7 g (0.4431 mol) of crystalline 2-butyl-4-chloro-5-formylimidazole (yield 66.4%). The analysis of this product by HPLC showed a purity of 92.5%, based on glycine. The yield and purity were comparable to those mentioned in example 4 of US 5,696,272 (62.0 % yield and 85 % HPLC purity), using a corresponding synthesis route and the same starting materials.

In order to arrive at the purity levels reported in example 2 (preparation of BCFI in the presence of a triflate catalyst), extensive purification, including a charcoal treatment, was required, accompanied by a drop in yield to about 50 %.

### Example 3 - Preparation of BFI from BCFI

In an autoclave 50 g (0.27 mol) of 2-butyl-4-chloro-5-formylimidazole was brought in 500 ml of methanol and 32 g of triethylamine was added hereto, followed by 2.5 g of 10% palladium on carbon. The hydrogen pressure in the autoclave was kept at 4 - 5 kg /cm² at 20 - 25 °C for 8 - 10 hrs, while monitoring the reaction by thin layer chromatography.

At the end of the reaction, the mixture was taken from the autoclave and the solvent was removed under reduced pressure below 50°C. 250 ml of deionised water was added to the dried mixture and it was cooled to 25-30°C. The pH was adjusted to 1.2 using diluted hydrochloric acid. The aqueous layer was then washed with 50 ml of dichloromethane to remove traces of the starting material. The pH was then readjusted to 6.8 - 7.5 using a sodium carbonate solution, and the aqueous layer was extracted with 3 x 150 ml of dichloromethane. Afterwards, the dichloromethane was dried with sodium sulfate for 30 min and then filtered to remove the sodium sulfate. This was followed by evaporation to dryness. 250 ml of hexane was added hereto at a temperature of 45 °C, and then cooled to 10 - 15 °C for 30 min, to obtain 2-butyl-5-formylimidazole.

The product was isolated by filtration followed by washing with 100 ml of chilled hexane (10 °C) and dried at 55 - 60 °C for 6 hrs. The yield of the dried material was 30 - 33 g (0.20 - 0.22 mol; 74 - 81 %). The analysis of this product by HPLC gave 99.1% purity, confirmed by IR.

### Example 4 - Preparation of BFI from valeronitrile

58 g (1.2 mol) of valeronitrile was charged in 34 ml of methanol and cooled to -5 to - 10 °C. Hydrochloric acid gas was slowly passed through for 15 - 18 hrs. The nitrogen pressure was kept at 1.5 to 2.0 kg/cm² for 14 hrs at 0 - 15 °C, followed by the addition of 32 ml of methanol while stirring for 60 min.

The reaction mass was transferred to a methanolic ammonia solution (12 - 15 wt% and stirred for 3 hrs at 20 - 30 °C, while keeping the pH at 8.0 - 9.0. Precipitated material was filtered off and washed with 15 ml of methanol. The filtrate was concentrated by distillation under reduced pressure (650 - 700 mm Hg) at a temperature not exceeding 90°C, followed by cooling to give pentanimidate.

50 g (0.666 mol) of glycine was added to a freshly prepared methanolic sodium hydroxide solution (sodium hydroxide 26.64 g (0.666 mol) in 250 ml of methanol) at 0°C and stirred for 15 min. The above prepared, 80 g pentanimidate was added at 0 - 5 °C in 10 - 15 min and the mixture was stirred for another 16 hrs at room temperature. The solvent was distilled under vacuum while keeping the temperature below 50°C.

500 ml of toluene was added to the above reaction mass, followed by 0.25 g of Copper(II) trifluoromethanesulfonate. Then 320 g (2.08 mol) of phosphorous oxychloride and 150 g (2.05 mol) N,N-dimethylformamide were added successively in 60 minutes and 2 hours, respectively. The reaction mixture was heated to 100 °C and stirred for another 2 hrs, then cooled to 30 °C and quenched in 260 ml of cooled water having a temperature below 25°C. 30 g of filter aid (hi-flow) was added and the pH adjusted to 1.2 with 440 ml of 30 % aqueous sodium hydroxide solution.

It was then filtered and washed with 100 ml of toluene, and the layers were separated. The toluene layer was washed twice with deionised water (400 ml each time), and 8 g of activated carbon was added to the toluene and stirred for 30 min at 30 - 35 °C, followed by filtration and washing with 100 ml of toluene. All toluene layers were combined and concentrated to dryness under vacuum below 55 °C. The concentrated mass was cooled to 30 °C. The weight of the residue was 96 g.

500 ml of methanol and 60 g of triethylamine was added hereto, followed by 4.5 g of 10% palladium on carbon in the autoclave. The hydrogen pressure was kept at 4 - 5 kg/cm² at 20 - 25 °C for 8 - 10 hrs, while monitoring the reaction by thin layer chromatography.

At the end of the reaction, the mixture was unloaded from the autoclave. The solvent was removed under reduced pressure, thereby keeping the temperature below 50 °C. 250 ml of deionised water was added and it was cooled to 25 - 30 °C. The pH was adjusted to 1.2 with diluted hydrochloric acid and the aqueous layer was extracted with 60 ml of dichloromethane to remove traces of the starting material. The pH was then readjusted to 6.8 - 7.5 using sodium carbonate solution, and the aqueous layer was extracted with 3 x 160 ml of dichloromethane. The dichloromethane solution was dried with sodium sulfate for 30 min and the sodium sulfate removed by filtering. The filtrate was evaporated to dryness, 300 ml of hexane was added at 45 °C, and the mixture was cooled to 10 - 15 °C and maintained at that temperature for another 30 min, to obtain 2-butyl-5-formylimidazole.

The product was isolated by filtration followed by washing with 150 ml of chilled hexane (10 °C) and dried at 55 - 60 °C for 6 hrs, with a yield of 58 g. Analysis of the product by HPLC showed 99.0 % purity. The yield was about 55 % on the basis of valeronitrile.

## Claims

1. A process for the preparation of a 2-substituted 4-chloro-5-formylimidazole of the formula: 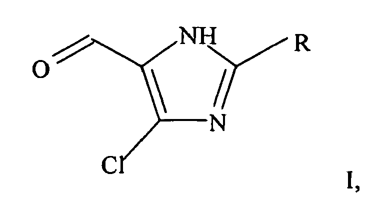 in which R is alkyl, alkenyl, cycloalkyl, arylalkyl or aryl, wherein glycine is reacted with an imido ester of the formula: 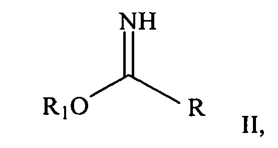 in which R has the meaning recited above, and R₁ is alkyl, to give a compound of the formula: 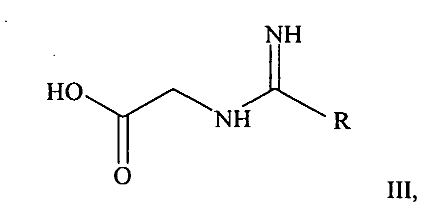 which is then subjected to a Vilsmeier reaction using a chlorinating agent and a formamide of the formula: 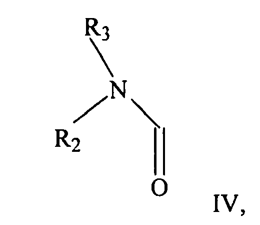 in which R₂ and R₃ are identical or different and each is a (C₁-C₄) alkyl, **characterized in that** said Vilsmeier reaction is performed in the presence of a triflate (trifluoromethane sulfonate) catalyst.

2. The process according to claim 1, wherein said imido ester II is prepared by reacting a nitrile having formula R-C≡N with methanol in the presence of hydrochloric acid gas, followed by a treatment with ammonia.

3. The process according to claim 2, wherein said imido ester II is not isolated and instead is further reacted directly with glycine.

4. The process according to any one of the preceding claims, wherein said 2-substituted 4-chloro-5-formylimidazole is 2-butyl-4-chloro-5-formylimidazole (R = butyl).

5. A process for the preparation of a 2-substituted 5-formylimidazole of the formula: 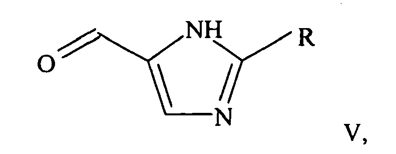 , in which R has the meaning as defined in claim 1,
by performing, the steps of the process according to any one of the preceding claims, to obtain the 2-substituted 4-chloro-5-formylimidazole of formula I, and subjecting said 2-substituted 4-chloro-5-formylimidazole to hydrodehalogenation in the presence of a noble metal catalyst.

6. The process according to claim 5, wherein said noble metal catalyst is palladium on carbon.

7. The process according to claim 5 or 6, wherein said 2-substituted 5-formylimidazole is 2-butyl-5-formylimidazole (R = butyl).

## Patentansprüche

1. Verfahren zur Herstellung eines 2-substituierten 4-Chlor-5-formylimidazols der Formel: 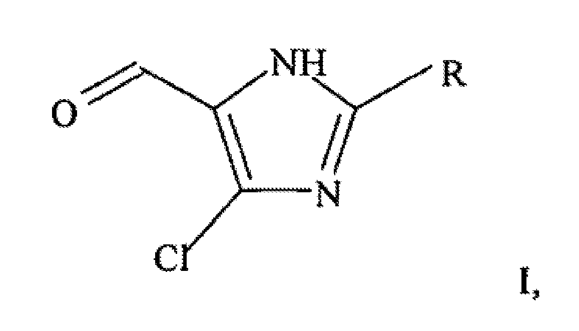 bei der R Alkyl, Alkenyl, Cycloalkyl, Arylalkyl oder Aryl ist, wobei Glycin mit einem Imidoester der Formel: 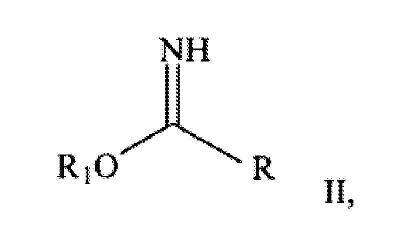 umgesetzt wird, bei der R die zuvor genannte Bedeutung hat, und R₁ Alkyl ist, um eine Verbindung der Formel: 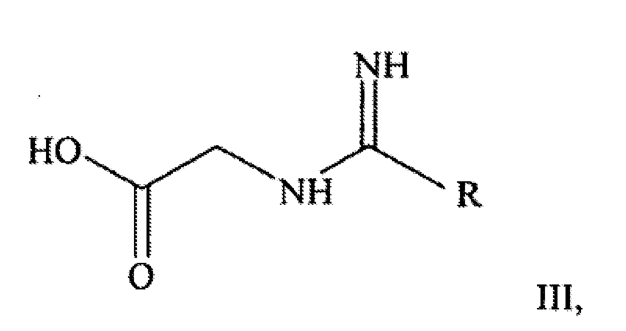 herzustellen, welche dann einer Vilsmeier-Reaktion unter Verwendung eines chlorierenden Mittels und eines Formamids der Formel: 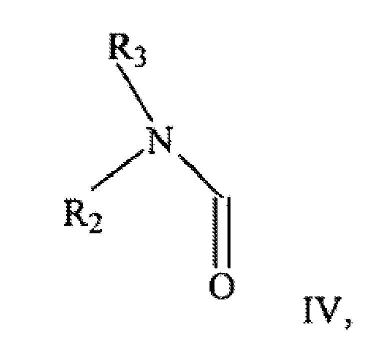 ausgesetzt wird, bei der R₂ und R₃ identisch oder unterschiedlich sind und jeweils ein (C₁-C₄) Alkyl ist, **dadurch gekennzeichnet, dass** die Vilsmeier-Reaktion in Anwesenheit eines Triflat (Trifluormethansulfonat)-Katalysators durchgeführt wird.

2. Verfahren nach Anspruch 1, wobei das Imidoester II hergestellt wird, indem ein Nitril mit der Formel R-G≡N mit Methanol in Anwesenheit von Salzsäuregas umgesetzt und anschließend mit Ammoniak behandelt wird.

3. Verfahren nach Anspruch 2, wobei der Imidoester II nicht isoliert wird und stattdessen weiter direkt mit Glycin umgesetzt wird.

4. Verfahren nach einem der vorhergehenden Ansprüche, wobei das 2-substituierte 4-Chlor-5-formylimidazol 2-Butyl-4-chlor-5-formylimidazol ist (R = Butyl).

5. Verfahren zur Herstellung eines 2-substituierten 5-Formylimidazols der Formel: 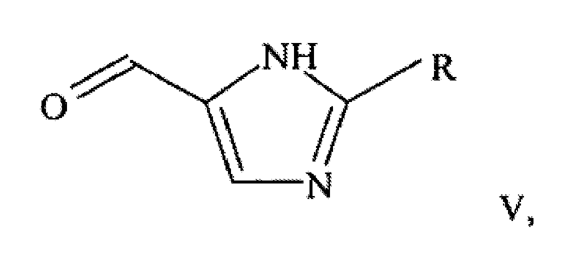 bei der R die Bedeutung hat wie in Anspruch 1 definiert, bei dem die Schritte des Verfahrens nach einem der vorhergehenden Ansprüche durchgeführt werden, um das 2-substituierte 4-chlor-5-formylimidazol der Formel I zu erhalten, und bei dem das 2-substituierte 4-chlor-5-formylimidazol einer Hydrodehalogenierung in Anwesenheit eines inerten Metallkatalysators ausgesetzt wird.

6. Verfahren nach Anspruch 5, wobei der inerte Metallkatalysator Palladium auf Kohlenstoff ist.

7. Verfahren nach einem der Ansprüche 5 oder 6, wobei das 2-substituierte 5-Formylimidazol 2-Butyl-5-formylimidazol ist (R = Butyl).

## Revendications

1. Procédé de préparation du 4-chloro-5-formylimidazole substitué en position 2 de formule : 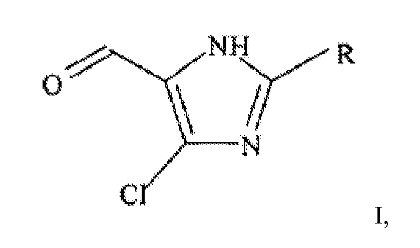 dans laquelle R représente un alkyle, un alcényle, un cycloalkyle, un arylalkyle ou un aryle,
dans lequel la glycine est mise à réagir avec un imido-ester de formule : 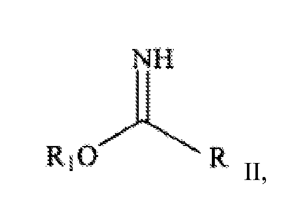 dans laquelle R a la signification donnée ci-dessus, et R₁ représente un alkyle, pour donner un composé de formule : 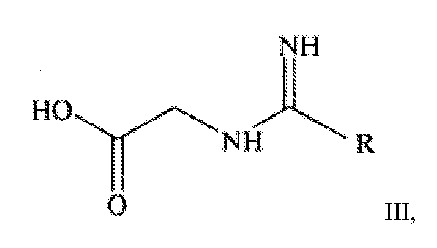 qui est ensuite soumis à une réaction de Vilsmeier à l'aide d'un agent de chloration et d'un formamide de formule : 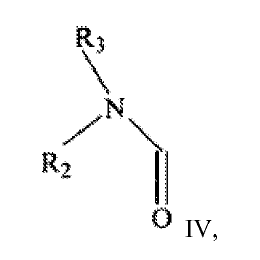 dans laquelle R₂ et R₃ sont identiques ou différents et représentent chacun un alkyle en C₁ à C₄,
**caractérisé en ce que** ladite réaction de Vilsmeier est effectuée en présence d'un catalyseur triflate (trifluorométhane sulfonate).

2. Procédé selon la revendication 1, dans lequel ledit imido-ester II est préparé en faisant réagir un nitrile ayant la formule R-C≡N avec du méthanol en présence d'acide chlorhydrique gazeux, suivi d'un traitement à l'ammoniac.

3. Procédé selon la revendication 2, dans lequel ledit imido-ester II n'est pas isolé et est plutôt mis à réagir directement avec la glycine.

4. Procédé selon l'une quelconque des revendications précédentes, dans lequel ledit 4-chloro-5-formylimidazole substitué en position 2 est le 2-butyl-4-chloro-5-formylimidazole (R = butyle).

5. Procédé de préparation du 5-formylimidazole substitué en position 2 de formule : 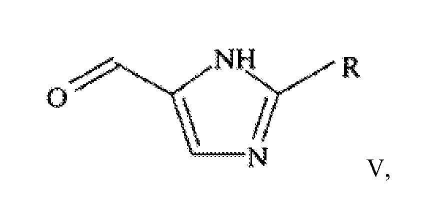 dans laquelle R a la signification telle que définie dans la revendication 1,
en effectuant les étapes du procédé selon l'une quelconque des revendications précédentes, afin d'obtenir le 4-chloro-5-formylimidazole substitué en position 2 de formule I, et en soumettant ledit 4-chloro-5-formylimidazole substitué en position 2 à une hydro-déshalogénation en présence d'un catalyseur à base de métal noble.

6. Procédé selon la revendication 5, dans lequel ledit catalyseur à base de métal noble est du palladium sur charbon.

7. Procédé selon la revendication 5 ou la revendication 6, dans lequel ledit 5-formylimidazole substitué en position 2 est le 2-butyl-5-formylimidazole (R = butyle).
